# EUROPEAN PATENT APPLICATION

(11) **EP 0 582 766 A1**
(43) Date of publication of application: **16.02.1994**
(21) Application number: 92830447.6
(22) Date of filing: 13.08.1992
(51) Int. Cl.: A61B 17/22, B06B 1/06

(54) **Ultrasonic recanalization system and transducer therefor**

(71) Applicant: MINISTERO DELL' UNIVERSITA' E DELLA RICERCA SCIENTIFICA E TECNOLOGICA, I-00196 Roma (IT)
(72) Inventor: Ercolani, Mauro, I-00154 Roma (IT); Jones, Hugh William R.R.2, 374 Viewmont Drive, Nova Scotia BOJ 3JO (CA); Klassen, Gerald Arthur, Nova Scotia B3H 2M5 (CA); Landini, Luigi, I-19038 Sarzana (La Spezia) (IT); Marraccini, Paolo, I-56100 Pisa (IT); Santarelli, Maria Filomena, I-56100 Pisa (IT)
(74) Representative: Bosotti, Luciano

(57) **Abstract**

An ultrasonic system for the recanalization of body vessels includes an ultrasonic transducer (3) adapted for in-situ insertion into body vessels, an ultrasonic power generator (1) as well as a highly insulated electrical cable (2) for conveying electrical power generated by said generator (1) towards said transducer (3) located in-situ into a body vessel. The ultrasonic transducer (3) is adapted for coupling to a catheter for guiding at the site of the occlusion in the vessel. Echoes from the occlusion are monitored, thereby permitting localisation of the transducer (3) in the body vessel.

## Description

### Field of the invention

The present invention relates to recanalization apparatus adapted e.g. for use in the treatment of vascular obstruction by atherosclerosis and its complications like thrombosis or embolism. The apparatus of the invention may also be employed to image differences in plaque composition such as soft versus calcified atheromata.

### Description of the prior art

Atherosclerosis is a disease of the arterial vessel wall which results in interference with blood flow to the body tissues. When obstruction occurs acutely in coronary arteries a myocardial infarction results (muscle cell death) or, if gradually, muscle ischemia (angina). Similar processes occur in other tissues when such conditions exist in the blood vessels in other parts of the body. Various techniques and devices may be employed in the treatment of atherosclerosis, thromboses and embolisms. The techniques known to date suffer from drawbacks that make an alternative procedure desirable.

Among these known techniques, the most commonly used to open vessels is balloon angioplasty, which involves the insertion of a catheter, having a circumferential balloon attached at its distal end, in the blood vessel. The catheter is slipped over a thin flexible guide wire and is advanced to the site of a stenosis. At this location, the balloon is inflated and thereby dilates the stenotic arterial segment. The limitations of balloon angioplasty are the high rate of early and late re-occlusion after peripheral and coronary angioplasty. Furthermore, most stenoses or occlusions cannot be treated by balloon angioplasty because such technique is not capable of opening the vessel especially in the presence of calcified blockages. In fact, very few non-surgical techniques are capable of opening up a calcified blockage. For example, a hot-tip catheter can be used for most fibrous blockages but cannot be used for totally calcified ones. Moreover, hot-tip catheters also have an increased risk of vessel walls perforation.

One of the alternatives to balloon angioplasty is laser-based angioplasty which dissolves or vaporizes the plaque. Laser angioplasty is limited by the high rate of arterial perforation due to the difficulty in controlling the depth of ablation.

The use of ultrasonic energy for vascular intervention has been shown to have potential for plaque ablation. In fact, it has been shown that acoustic energy is able to destroy atherosclerotic plaque and thrombi while leaving the underlying healthy vascular endothelial tissue undamaged.

A number of methods have been proposed for ultrasound angioplasty. Exemplary of such methods are, e.g. EP-A-0 316 796, EP-A-0 443 256, W0-A-89/06515, WO-A-90/01300 or US-A-4 808 153.

Experience with ultrasonic drill techniques has demonstrated that healthy vascular tissue is particularly resistant to ultrasonic energy, while the plaque is effectively drilled or reamed out. Moreover, techniques based on vibrating wire probe were proposed to open blood vessels that are totally occluded by atherosclerotic and/or calcified plaque.

Nevertheless, such techniques and devices still suffer from drawbacks. In fact, all such apparatus provide for ultrasonic power generation external to the patient's body, so that the rotational or vibrational energy is brought to the site of occlusion through a wire that rotates or vibrates along the vessel.

A number of drawbacks limit such ultrasound systems for percutaneous angioplasty. First, since the ultrasound generator must be located outside the body, it is necessary, particularly for coronary applications, to transmit vibratory energy over long distances ranging from 60 to 100 cm. Consequently, attenuation of ultrasound energy along the transmission wire results in a loss of efficiency for the system which ultimately results in a remarkable reduction of the energy delivered to the arterial site under treatment.

Secondly, attenuation of ultrasound energy produces heat. Such heat production rapidly increases the temperature of the transmission wire, with serious effects for patient safety.

Still another problem with such percutaneous techniques is the danger of perforation of the vessel wall both during the advancing in the blood vessel and during application of ultrasonic energy in-situ. The wire which is transmitting the ultrasound energy may be bent and be in firm contact with a blood vessel at points where treatment is not intended; this may cause the blood vessel to be damaged or perforated.

### Object and synthesis of the invention

The underlying problem of the present invention is providing a system for ultrasonic angioplasty which overcomes the drawbacks outlined in the foregoing. According to the invention, such problem is solved by means of a system having the characteristics set forth in claim 1. Advantageous developments of the invention are called for in the subclaims.

In brief, the present invention provides, in the preferred embodiment, an ultrasonic system for angioplasty which includes an ultrasonic transducer adapted to be located in-situ in the region of the body vessel under treatment, a highly insulated electrical cable containing electrical wires required for conveying electrical power to the ultrasonic transducer (angioplasty mode) and for receiving the echoes from the occlusion (monitoring mode), a catheter for guiding the ultrasonic transducer to the site of the occlusion in the body vessel, and an ultrasonic power generator and an electronic pulse receiver, both external to the patient.

The ultrasonic system of the invention overcomes the problems of heating and perforation exhibited by the solutions of the prior art, since vibrational energy is produced directly in-situ (i.e. at the site of the occlusion) by the ultrasonic transducer; heating is avoided because of the high efficiency of the ultrasonic transducer, so that electrical energy is mainly converted into vibrational energy.

### Detailed description of the invention

The invention will now be described, by way of non-limiting example, with reference to the enclosed drawings, in which:
figure 1 is a block diagram showing the overall structure of the system of the invention when used in a typical angioplasty mode;
figure 2 is another block diagram showing the structure of the system of the invention adapted for use also in a monitoring mode;
figure 3 is a graph showing the time behaviour of some signals generated in the system of the invention, and
figures 4 to 11 show various embodiments of transducers adapted for use in the system of the invention.

Essentially, a system according to the invention is comprised of an ultrasonic generator 1 which generates an electrical power signal for feeding, through a highly insulated electrical cable 2, an electromechanical transducer 3 for converting the electrical power produced by generator 1 into an ultrasonic vibratory wave.

Generator 1 is of a kind which is well known in the art (see e.g. the various patent documents referred to in the introductory portion of the description), whereby no detailed description of the structure and operation thereof will be provided here. Preferably, generator 1 has associated therewith a tuning circuit 4 (likewise of known type) for selectively controlling oscillation of the generator 1 itself in order to vary the operating frequency thereof and/or to keep it substantially constant irrespective of possible variations induced by changes in the impedance of transducer 3 during operation.

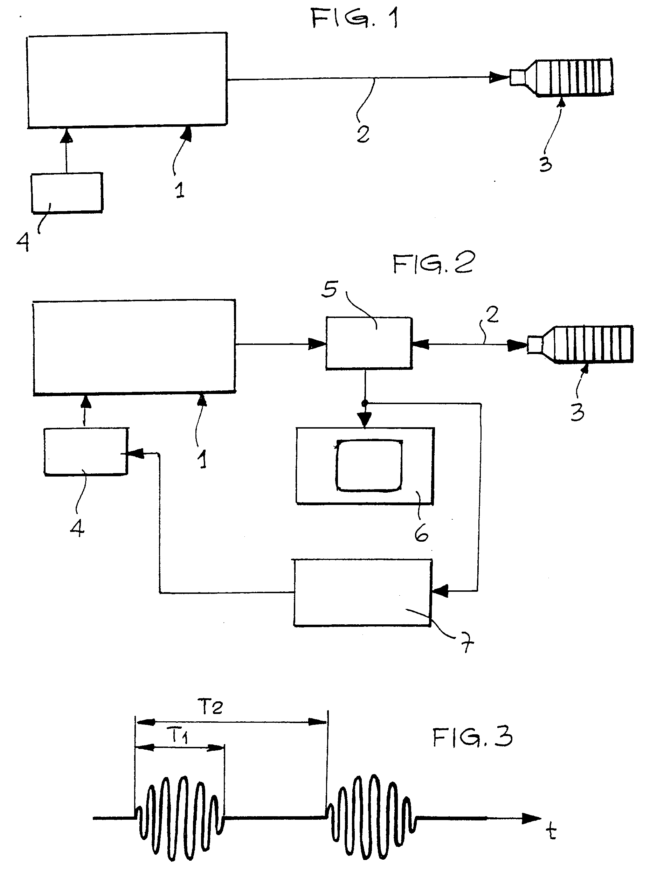

In the monitoring arrangement of figure 2, an electronic pulse receiver circuit 5 is provided (which can be easily included in the generator 1 as a time window gating function) which senses any echo signals received back from the transducer 3 along cable 2 and feeds them to a display unit 6 which provides an indication (in a typical echographic arrangement) of the location of the transducer 3 with respect to the occlusion to be treated.

A feedback unit 7 can also be provided for sensing, based on the signal received from unit 5, any variation of the impedance of transducer 3 in order to control the tuning circuit 4 to keep constant the oscillating frequency of generator 1.

The range of the overall diameters of the ultrasonic transducer 3 is preferably from about 1.0 mm to about 5 mm in diameters with lengths from about 2.5 mm to about 20 mm. Such dimensions, particularly diameters between about 1.0 mm and about 2 mm and lengths from about 2.5 mm to about 10 mm make the invention particularly appealing in coronary applications. In fact, it would also be widely used in patients for in-situ treatment of acute coronary syndromes to disrupt and liquify clot and for concurrent removal of obstructive lesions. To that effect, cable 2 can be incorporated to a catheter for guiding the transducer 3 at the site of the occlusion.

In the angioplasty mode (figure 1) the ultrasonic power generator 1 is preferably operated at frequencies in the range of 35 KHz to 1.5 MHz. The ultrasonic power generator 1 has variable a duty cycle (T1, T2 - see figure 3) to facilitate generation of pulsed ultrasound. Also, the ultrasonic power generator 1 is capable of delivering an output power in the range from about 5 to about 10 Watts per cm² when driven from a matching source with a maximum output voltage of about 100 Vrms. The power generator 1 has variable power output in order to operate in the presence of a wide variety of plaque compositions.

Generally, these acoustic power outputs are obtained with an applied voltage of 60 Vrms or less. The tuning circuit 4 ensures matching so that the maximum power is transferred to the load (occlusion). Such operation is extremely important especially when dealing with occlusions having inhomogeneous compositions.

In the monitoring mode (figure 2) the system of the invention also provides for ultrasonically monitoring the vessel occlusion. This is accomplished with the same ultrasonic transducer 3 employed for angioplasty by using the transducer 3 itself in a pulsed mode as an echo sounder (see again figure 3) to detect lesions and sense their dispersion. For the monitoring mode the ultrasonic generator duty cycle is set in order to deliver ultrasonic energy to the ultrasonic transducer 3 for a small time, preferably from 2 µs to 20 µs. Such operation is accomplished by changing the duration T1 of the active portion of the duty cycle; after that time, the ultrasonic transducer 3 detects echoes from the vessel lesion and through the electrical wire 2 the echo signals are sent towards the electronic pulse receiver circuit 5 which amplifies the echoes suitably for display. Display operation may be accomplished in 6 by using a cathode ray tube (CRT) based oscilloscope. For monitoring operation echo signals range from 1 mV to 100 mV.

The ultrasonic transducer 3 is encapsulated in a sheath 8 of a material which is biocompatible and does not induce clotting. The preferred encapsulation is non-porous, high dielectric strength material like polymers, epoxy resins, silicone. The encapsulation 8 may ensure suitable impedance matching between the ultrasonic transducer 3 and the biological tissue in order to allow the maximum power transfer to the tissue.

In operation, ultrasound energy generated in-situ by the transducer 3 if focussed so that the highest ablation energy occurs in dense tissue within the vessel wall such as plaque or intervascular clot while living cells such as endothelium and smooth muscle are not affected. For biological purposes, the maximum power output of the ultrasonic transducer 3 is preferably confined to a distance of no more that 1 cm from the transducer. The ultrasonic power falls off rapidly with increasing distance from the tip of the transducer. Lysis occurs through a process of microcavitation whereas the vessel is gently opened by intravascular pressure rather than by a balloon with its accompanying vessel wall trauma as is the case with balloon angioplasty.

The success of the intervention can be immediately evaluated by imaging the vessel and measuring the restored blood flow by devices (not shown) which can be mounted on the catheter supporting the ultrasonic power transducer 3. Preferably, the ultrasonic transducer 3 is proposed for coronary angioplasty. As a by-product the transducer 3 can also be used in peripheral arteries to restore blood flow when the vessel has been occluded by embolus, clot or atheromata.

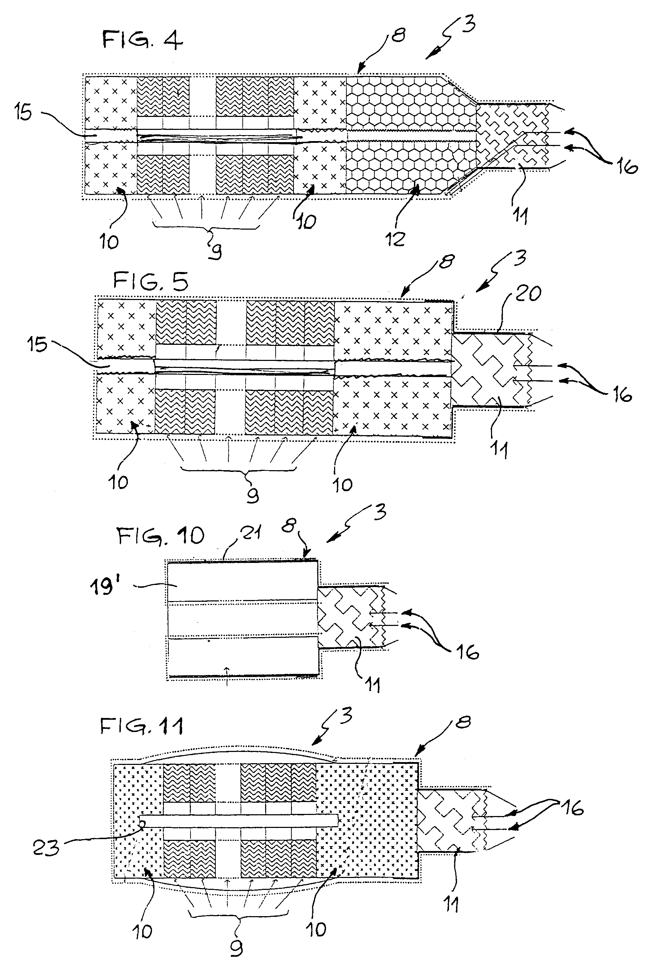

Figures 4 through 11 show a number of different possible types of ultrasonic transducers 3 adapted for mounting on catheters.

Specifically, the transducer types of figures 4, 5, 10, and 11 provide for end-on mounting of the transducer 3 at the distal end of a catheter which can incorporate the cable 2 for conveying electrical signals to and from transducer 3.

Although the figures show the catheter tube (labelled 11) smaller in diameter than the transducer 3, this may be the case on the larger diameter transducers only, the smaller transducers having diameters equal to or less than the catheter tube.

The other transducer types shown provide hollow type transducers having a tubular structure overall, thereby permitting the transducer to slide on a guide wire C. The characteristics of the transducers fall into a range of values which is decided by their size and constructional type.

Generally speaking, the intrinsic resonance frequency of the transducer is chosen (by resorting to end loads as better explained in the following) in order to confine it below 1 MHz. At least for some transducer types such resonance frequency can be chosen so that the transducer 3 generally behaves as a λ/2 resonator, λ being the wavelength of the ultrasonic waves generated.

Figures 4 through 7 show transducers wherein the ultrasonic electromechanical converter means are a stack of piezoelectric ceramic rings 9 having associated therewith end loads 10.

Suitable materials for the construction of ultrasonic rings 9 are PZT 4/5a produced by Morgan Matroc - Vernitron Division Ltd. (Thornhill, Southampton, U.K.), but other piezoelectric ceramics with good high-drive characteristics can be used successfully.

The electrical and acoustical coupling between adjacent ceramics rings 9 in the stack is obtained by interposing a metal loaded conductive glue. The conductive glue sold under the trade name Elecolit by Panacol-Elosal GmbH (Frankfurt, Germany) was found to provide fully satisfactory results both in terms of electrical coupling between adjacent ceramics 9 and as regards establishing wire connections with the electrical power generator 1.

The piezoelectric ceramics rings 9 in the stack are electrically connected to the power generator 1 in a parallel configuration in order to force all the ceramic rings to operate in a "phase mode". In that way, the excitation signals conveyed towards the transducers 3 can be significantly reduced down to values of less than 100 Vrms, which appears to be largely compatible with the need of supplying electrical power to the site of the occlusion. This is typically well inside the patient's body and, more to the point, in areas, such as the heart region (for coronary angioplasty), where electrical signals may possibly interfere with physiological signals such as stimulation of the heart muscle.

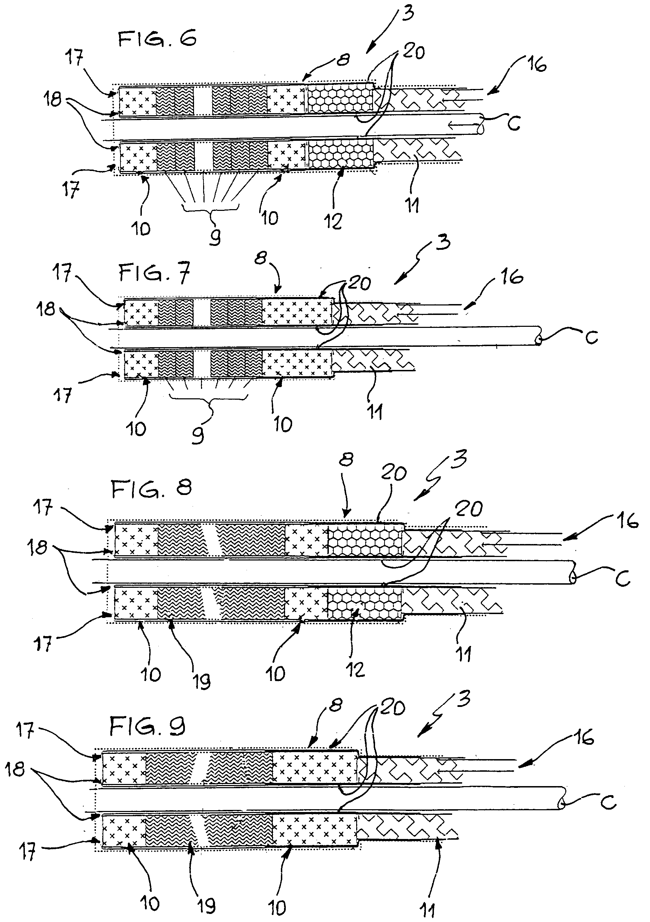

In the transducers shown in figures 8 and 9 the ultrasonic electromechanical converter is comprised of a cross-polarized tubular piezoelectric element designated 19.

In either case, the end loads 10 are intended to reduce the intrinsic resonance frequency of the transducer 3 to the values mentioned in the foregoing.

The end loads 10 may be symmetrical end loads (figures 4, 6 and 8) having a pad of pressure release type material labelled 12 at the (proximal) end of the transducer 3 closest to the catheter tube 11.

In that way, the almost symmetrical ultrasonic wavefront generated by ceramics 9 is dampened by pad 12 on the rear (proximal) side of the transducer, whereby most of vibratory energy is radiated from the front (distal) side, which in use is directed towards the occlusion. Alternatively, in figures 5, 7, and 9 asymmetrical end loads 10 are shown having a large load which terminates the transducer at the catheter tube end at a point of infinite acustical impedance located at the proximal end of the transducer 3. Consequently, most of the ultrasound energy is radiated from the opposite, distal end of the transducer.

The resonance frequency of the ultrasonic transducer 3, is a function of the overall transducer length (including ceramics 9 in the stack and end loads 10).

A tensioning element 15 in the form of a bar is provided in the transducer shown in figures 4 and 5 in order to apply an axial constraint to the stack of piezoelectric rings 9. Element 15 may be comprised of a rod of metal or Kevlar or other suitable fibre element. Preferably, the tensioning element 15 is attached to the end loads 10 by screw threads, but one of several fastening techniques can be used which may include shrink fits, swaging, glues or settings using molten metal assemblies.

As indicated, figures 6 through 9 show tubular transducers designed to accept guide catheters/wires (labelled C) of diameters which are used in current medical practice.

The transducers shown in figures 6 through 9 are modified Tonplitz transducer types which have their tensioning element(s) in the form of one or two tubular element(s) labelled 17 and/or 18 i.e. as tubular linings of the outer/inner surfaces of the piezoelectric element(s) 9; 19 having respective end portions wrapped around the piezoelectric element(s) in order to ensure axial constraint thereof. If the external element 17 is used, then the inner element 18 is a plastics or similar tube to accomodate the guide wire.

As indicated figures 8 and 9 show transducer basically corresponding to those of figures 6 and 7 in which the stack of piezoelectric rings 9 is replaced by the cross-polarized tubular element indicated 19. In other respects i.e end loads 10 (symmetrical or asymmetrical), pressure relief and tensioning elements 17, 18, these latter transducers are thoroughly similar to the transducers which have been described earlier.

Wires for supplying electrical excitation energy to ceramics 9, 19 are labelled 16 in the figures and are connected to a parallel net in the piezoelectric element(s).

The transducers 3 are tethered to the catheter tube by a wire or fibre element (see figures 1 and 2) or by a tubular tether designated 20 in figures 5 through 8; in the latter case the tether may be attached to the inside surface or the outside surface of the transducer or to the end load. The transducers are incapsulated by the sheath 8 and therefore exhibit non-thrombotic surfaces to the patient.

Figures 10 and 11 show other configurations complementary to those previously described. In other respects, i.e. end loads, symmetry, pressure relief and tensioning elements, they are similar to the transducers which have been described earlier.

Figure 10 shows a hollow tubular transducer 19' which operates in the radial mode and is under pressure from a peripheral wire or fibre wrapping labelled 21. Figure 11 shows a barrel stave transducer which operates in a bending mode; part label led 23 is an element which applies compression to the piezoelectric stack which is labelled 9.

The catheter assemblies may include other equipment required for diagnostic and treatment purposes as is the practice in cardiovascular treatment and exploration. Particularly, the catheter assembly may contain an element to indicate the acoustical power levels generated by the transducer. Of course, the piezoelectric rings 9 or the tubular element 19, 19' can be replaced by other kinds of electromechanical converter means.

## Claims

1. An ultrasonic recanalization system including means (1, 3) for generating ultrasonic waves to be applied in-situ to effect recanalization of body vessels, characterised in that it includes:
- an electromechanical ultrasonic transducer (3) adapted for in-situ insertion into body vessels,
- an ultrasonic power generator (1) for generating an electrical signal for driving said transducer (3), and
- electrical power conveying means (2) for connecting in use said ultrasonic power generator (1) located outside the body to said transducer (3) located in-situ into a body vessel.

2. A system according to claim 1, wherein said ultrasonic power generator (1) has operating frequencies in the range of 35 KHz to 1.5 MHz.

3. A system according to claim 1 or claim 2, characterised in that said ultrasonic power generator (1) generates a pulse electrical signal (T1, T2).

4. A system according to claim 3, characterised in that said pulse electrical signal has a variable duty cycle (T1,T2).

5. A system according to any of the preceding claims, characterised in that said ultrasonic power generator (1) has a variable power output.

6. A system according to any of the preceding claims, characterised in that said ultrasonic power generator (1) has associated tuning means (4) for selectively controlling the frequency of said electrical signal.

7. A system according to claim 1, characterised in that said ultrasonic power generator (1) delivers a maximum output voltage of about 100 Volts rms.

8. A system according to claim 1, characterised in that said ultrasonic power generator (1) delivers an output voltage of about 60 Volts rms.

9. A system according to any one of claims 1 to 8, characterised in that it further includes monitoring means (5, 6) for monitoring energy reflected back from said transducer (3) towards said ultrasonic power generator (1) to effect localisation of said transducer (3) within said body vessels.

10. A system according to claims 6 and 9, characterised in that said monitoring means include a feedback unit (7) for varying the operating frequency of said ultrasonic power generator (1) according to the location of said transducer (3) into said body vessel.

11. An electromechanical ultrasonic transducer (3) including electromechanical converter means (9;19;19') adapted for in-situ insertion into body vessels to convert electrical power into an ultrasonic wave to be applied in-situ to effect recanalization of body vessels.

12. A transducer according to claim 11, characterised in that it has an overall diameter from about 1.0 mm to about 5 mm, preferably from about 1.0 mm to about 2 mm.

13. A transducer according to claim 11, characterised in that it has a length from about 2.5 mm to about 20 mm, preferably from about 2.5 mm to about 10 mm.

14. A transducer according to claim 11, characterised in that it includes a stack of piezoelectric elements (9).

15. A transducer according to claim 11, characterised in that it includes a tubular piezoelectric element (19).

16. A transducer according to claim 14, characterised in that said piezoelectric elements in the stack are in the form of rings (9).

17. A transducer according to claim 14 or claim 16, characterised in that said piezoelectric elements (9)in the stack are electrically connected in a parallel configuration.

18. A transducer according to claim 14 or claim 16, characterised in that said piezoelectric elements (9) in the stack have interposed therebetween a conductive layer.

19. A transducer according to claim 18, characterised in that said conductive layer is comprised of a conductive glue.

20. A transducer according to any one of claims 11 to 19, characterised in that said electromechanical converter means (9; 19) has associated therewith end loads (10) to reduce the intrinsic resonance frequency of said electromechanical converter means (9; 19).

21. A transducer according to claim 11 or claim 20, characterised in that the transducer (3) has an intrinsic resonance wavelength corresponding to λ/2, where λ is the wavelength of the generated ultrasonic waves.

22. A transducer according to claim 20 or claim 21, characterised in that said end loads (11) are substantially symmetrical, the end load (10) associated to the proximal end of said transducer (3) in use having associated therewith ultrasonic energy dampening means (12), whereby ultrasonic energy is radiated primarily from the distal end of said transducer (3).

23. A transducer according to claim 20 or claim 21, characterised in that said end loads (10) are asymmetrical with a larger load associated with the proximal end of said transducer (3) in use in order to form a point of infinite acoustical impedance, whereby ultrasound energy is radiated primarily from the distal end of said transducer (3).

24. A transducer according to any one of claims 11 to 23 characterised in that said electromechanical converter means (9;19;19') has associated therewith a constraint generating means (15; 17, 18; 21; 23).

25. A transducer according to claim 24, characterised in that said constraint generating means (15; 17, 18) applies an axial constraint to said electromechanical converter means (9).

26. A transducer according to claim 24, characterised in that said constraint generating means (23) applies a bending force to said electromechanical converter means (9).

27. A transducer according to any one of claims 24 to 26, characterised in that said constraint generating means is in the form of at least one rod (15).

28. A transducer according to claim 27, characterised in that said at least one rod (15) is made of a material selected from the group consisting of metal and fibres.

29. A transducer according to claim 24, characterised in that said constraint generating means (17, 18) are in the form of at least one tubular member surrounding and/or inserted into said electromechanical converter means (9; 19).

30. A transducer according to claim 24 or claim 29, characterised in that said constraint generating means are in the form of a wire or fibre wrapping (21) around said electromechanical converter means (19').

31. A transducer according to any one of claims 11 to 30, characterised in that it is connected to one end of a catheter (11).

32. A transducer according to any one of claims 11 to 31, characterised in that it has a tubular structure overall having an axial opening to allow passage of a wire-like guiding element (C).
